# EUROPEAN PATENT APPLICATION

(11) **EP 1 541 579 A1**
(43) Date of publication of application: **15.06.2005**
(21) Application number: 03760921.1
(22) Date of filing: 20.06.2003
(51) Int. Cl.: C07H 15/203, A61K 31/7028, A61P 11/00, A61P 11/06, A61P 11/08, A61P 43/00, C07H 15/18

(54) **PRODURG, MEDICINAL UTILIZATION THEREOF AND PROCESS FOR PRODUCING THE SAME**

(30) Priority: 20.06.2002 JP 2002180238
(71) Applicant: Nippon Suisan Kaisha, Ltd., Tokyo 100-0004 (JP)
(72) Inventor: YAMASHITA, Shinya, c/o Nippon Suisan Kaisha, Ltd., Hachioji-shi, Tokyo 192-0906 (JP); TAKEO, Jiro, c/o Nippon Suisan Kaisha, Ltd., Hachioji-shi, Tokyo 192-0906 (JP); OKITA, Takaaki, c/o Nippon Suisan Kaisha, Ltd., Hachioji-shi, Tokyo 192-0906 (JP)
(74) Representative: VOSSIUS & PARTNER
(86) International application number: PCT/JP2003/007868
(87) International publication number: WO 2004/000863

(57) **Abstract**

A prodrug utilizes an enzyme whose enzymatic activity is different in between the target site of the drug and the site to express side effects, the prodrug having a substituent cleavable with the enzyme and being activated by cleaving the substituent with the enzyme. As the target site of the drug, for example, a respiratory organ can be mentioned and as the site to express side effects, for example, the heart can be mentioned. As the example of the drug, a bronchodilator can be mentioned and as the example of the enzyme, a glycosidase (for example, β-glucuronidase) can be mentioned. Furthermore, the substituent is, for example, a glycosyl group composed of a monosaccharide or an oligosaccharide. Use of the enzyme enables reducing the side effects of a drug of the type whose target site is different from the site to express side effects.

## Description

### TECHNICAL FIELD

The present invention relates to a produrg which can reduce the side effects of the drug by utilizing an enzyme whose enzymatic activity has a difference in between the target site of the drug and the site to express side effects.

### BACKGROUND ART

A number of so-called sugar substituent-bonded prodrugs have been studied. Their major object is to improve the solubility of the difficultly soluble parent compounds and to render them nontoxic on the analogy of a glucuronic conjugate. Particularly, the latter utilizes the metabolism of the body of living. In other words, the prodrugs are designed based on the thought that undesirable side effects are reduced while allowing the parent compounds to express their effect only at the affected part on the basis of the reports that the activities of sugar cleavable enzymes such as β-glucuronidase and β-glucosidase in the cancer cell and the inflammatory cell are increased. Their details will now be explained below.

Research reports that the activities of several glycosidases including β-glucuronidase are accelerated in the tumor tissue are published (Fishman, Science, 105, 646-647, 1947, Fishman and Anlyan, Cancer Res., 7, 808-814, 1947, and Bollet et al., J. Clin. Invest., 38, 451, 1959). With other disorders, it is reported that in an asthmatic patient the β-glucuronidase activity in the alveolar lavage fluid (BALF) by the liberation of β-glucuronidase from the alveolar macrophage and the mastocyte trends to be accelerated (Tonnel et al., Lancet, 8339, 1406-1408, 1983 and Murray et al., N. Engl. J. Med., 315, 800-804, 1986), and further it is reported that the β-glucuronidase and N-acetyl-D-glucosami-nidase activities are accelerated in the synovial fluid of a rheumatic patient (Stephens et al., J. Rheumatol., 2, 393-400, 1975), and the β-glucuronidase activity in the serum of an AIDS patient is high compared to the healthy individual and the like (Saha et al., Clin. Chim. Acta., 199, 311-316, 1991), and thus in the affliction of various types of disorders the acceleration of the activity of a glycosidase or the extracellular liberation of a glycosidase is suggested.
Of these glycosidases, β-glucuronidase of an especially noted enzyme is an enzyme which hydrolyzes a β-glucuronide to catalyze the reaction to liberate D-glucuronic acid, and it is reported that β-glucosidase is present in a wide range of organs such as the liver, the lungs, the spleen, and the kidneys or inflammatory cells such as macrophages and eosinophils (Hayashi, J. Histochem. Cytochem., 15, 83-92, 1967 and Conchie et al., Biochem. J. 71, 318-325, 1959).

In the chemotherapy of cancers an important problem is to reduce the toxicity against the normal tissue or the normal cell other than the tumor. In order to solve this problem, a number of antitumor agents which specifically act on the tumor tissues were developed but the expected reduction of side effects has not been found in any of them.

De Duve took note of a hydrolase in the lysosome containing a glycosidase in the tumor tissue and proposed the concept of the chemotherapy by the prodrug of an antitumor agent to be activated by the hydrolysis with the hydrolase and the enzyme (Biological approaches to cancer chemotherapy, 101-112, Academic Press, Inc., 1961).
Connors and Whisson showed in the experiment using mice a high correlation between the antitumor effect of aniline mustard of an antitumor agent and the β-glucuronidase activity of the tumor cell (Nature, 210, 866-867, 1966). Sweeny et al. published a theory about the mechanism of the action of mycophenolic acid of an antitumor agent that mycophenolic acid is glucuronidated in an organ and the resulting glucuronic conjugate is hydrolyzed with β-glucuronidase in the tumor tissue to form an active form of mycophenolic acid which exhibits the antitumor effect (Cancer Res., 31, 477-478, 1971). Young et al. conducted a clinical test with a cancer patient on the hypothesis that aniline mustard of an antitumor agent is glucuronidated within the body and the resulting glucuronic conjugate exhibits the antitumor effect by the hydrolysis in the cancer tissue as in the case of mycophenolic acid of an antitumor agent but a sufficient correlation between the antitumor effect and the enzymatic activity was not recognized (Cancer, 38, 1887-1895, 1976). Baba et al. reported that with the use of a mouse breast cancer model, a glucuronic acid derivative of 5-fluorouracil of an antitumor agent is intravenously administered to exhibit an inhibition (Gann, 69, 283-284, 1978). However, the sugar derivative prodrugs of these antitumor agents are generally insufficient in the hydrolysis in the target site, and accordingly satisfactory results of their clinical test have not been obtained.

Next, an approach was made that a product obtained by bonding a tumor-specific antibody to various enzymes was administered beforehand and a prodrug which was to be converted to the active form by the cleavage with these enzymes is used. This is called ADEPT (antibody-directed enzyme prodrug therapy) and a number of researches and developments were done but there are problems that an exogenous antibody-enzyme composite has immunoantigenicity and the prodrug cannot be sufficiently activated within the body of living, and thus the ADEPT has not obtained success yet.

Then, Bossler et al. (Br. J. Cancer, 65, 234-238, 1992) synthesized a compound through a spacer without directly bonding a sugar to an antitumor agent having low immunoanti-genicity in order for the administered prodrug having an antitumor agent-sugar derivative structure to efficiently undergo hydrolysis in the cancer cell and tried to improve the above described problem. In this process they found derivatives which exhibited a sufficient effect with glycoside-spacer derivatives alone and disclosed structures of glycoside-spacer drugs as the prodrugs applicable to antiinflammatories, immunosuppressives, calcium antagonists, sympathomimetic substances and the like in addition to antitumor agents {U.S. Patent No. 5,621,002 (Family Patent: European Patent Application Publication No. 642799, Japanese Patent Publication No. Hei 7-149667/1995), U.S. Patent No. 5,935,995 [Family Patent: European Patent Application Publication No. 795334, Japanese Patent Publication (Kokai) No. Hei 10-1495/ 1998], and U.S. Patent No. 5,955,100 [Family Patent: European Patent Application Publication No. 595133, Japanese Patent Publication (Kokai) No. Hei 6-293665/1994]}.

In Japanese Patent Publication (Kokai) No. Hei 6-293665/1994, it is described that "the compounds are activated by enzymes which in the healthy individual occur principally inside cells but which under the abovementioned pathophy- siological conditions have a local extracellular occurrence" and "The prodrugs according to the invention can be employed for all non-oncological disorders in which macrophages, granulocytes and platelets occur, especially in the activated state. In the activated state, the abovementioned cells mainly secrete intracellular enzymes which make site-specific activation of the prodrugs according to the present invention possible."

Further, it is described that on the basis that the substances of this citation in the case of using an antitumor agent as the active drug are not only recognized in the tumor model but also have been recognized in several inflammatory models, they can be supposed for all disorders in which inflammatory cells participate as in tumor.

The illustration of the drugs extends to "a cytostatic, an antimetabolite, a substance which intercalates into DNA, a drug which inhibits topoisomerase I+II, an alkylating agent, a compound which inactivates ribosomes, a tyrosine phospho-kinase inhibitor, a differentiation inducer, a hormone, a hormone agonist, a hormone antagonist, a substance which alters the pleiotropic resistance to cytostatics, a calmo-dulin inhibitor, a protein kinase C inhibitor, a p-glyco-protein inhibitor, a hexokinase modulator, an inhibitor of p-glutamylcysteine synthetase or of glutathione S-tranferase, an inhibitor of superoxide dismutase, an inhibitor of proli-feration-associated protein, a substance which has immuno-suppressant effects, a non-steroid antiinflammatory substance, an antirheumatic drug, a steroid, a substance which has anti-inflammatory, anlgesic or antipyretic effect, a derivative of an organic acid, an analgesic agent, a local anesthetic, an antiarrhythmic, a Ca-antagonist, an antihistaminic, an inhibitor of phosphodiesterase, a parasympathomimetic, a sym-pathomimetic, and a substance with an inhibitory effect on human urokinase".

In other words, the prodrugs of this citation are suggested to have a possibility of functioning in all drugs in which inflammatory cells participate but as to which drug actually functions and which drug does not actually function, no index is suggested.

In the Examples, only the measurements of the antitumor effect of the sugar derivatives of doxorubicin, nitrogen mustard, and quinine and those of the antiinflammatory effect and the acute toxicity of the doxorubicin sugar derivatives are shown, and in spite of the enumeration of therapeutic drugs described as above, no example is described showing the concrete drug effect and pharmacological action of the drugs other than antitumor agents, and thus it can be thought that their actual effectiveness has not been confirmed yet. For example, many studies on sugar derivatives of steroids have heretofore been made but as indicated by Sugai et al. (WO95/09177), with the problems of their safety and the expression of side effects by the conversion to active forms by enzymatic hydrolysis in organs other than the target organ, their development is in a difficult situation. Even doxorubucin which has been long studied is still in the preclinical stage in Europe and has not clinically been completed.

Furthermore, as other applications than to cancers, the study of the prodrugs using the glycosides of the above described steroids has been developed from early in order to reduce side effects. In 1962, 1964, and 1966, a group of Merck Co. showed a possibility for glycoside derivatives of steroids to reduce the side effects of the steroids such as adrenal atrophy, weight loss, osteoporosis, the reduction of the leukocyte count (British Patent Nos. 1015396 and 1059548, U.S. Patent No. 3,185,682, and Hirshmann et al., J. Am. Chem. Soc., 86, 3902-3904, 1964). However, the problem that the glycoside prodrugs of steroids have extremely inferior stability and the glycosidic bond is cleaved in other sites than the target site to express side effects became clear, and Sugai et al. (WO95/09177) tried to reduce the side effects. However, clinical success has not been obtained yet. Steroids can exhibit their effect in trace amounts and have variegated physiological effects in a wide range of tissues in the body of living, and accordingly can be said very difficult drugs to achieve the reduction of side effects.

Furthermore, Friend et al. took notice of the glycosidase which intestinal bacteria possessed and made investi-gations of the prodrugs having a sugar derivative structure of a steroid which cause a problem of side effects as the therapeutic drugs for ulcerative colitis {European Patent Application Publication No. 123485 [Family Patents: Japanese Patent Publication (Tokuhyo) No. Sho 60-501105/ 1985]}, J. Med. Chem., 27, 261-266, 1984, J. Med. Chem., 28, 51-57, 1985, and Pharmaceutical Res., 10, 1553-1562, 1993). However, these trials have not shown a clinical success up to now.

As described above, the development of prodrugs of drug-sugar derivatives which utilize the enzymes of the body of living including β-glucuronidase has been tried for a long time and has not been tied in with clinical success under the present conditions.

As the therapy of asthma which is one of the disorders of respiratory organs whose patients are great in number, a therapy of jointly using a long-acting β₂-agonist and an inhalation steroid is recommended according to the present guideline.

The history of the development of bronchodilators of β₂-agonists evolves from β-agonists which act on both the originally discovered β₁ and β₂ receptors into short-acting β₂ selective agonists of the second generation represented by salbutamol and develops into long-acting β₂ selective agonists of the third generation represented by salumeterol. The short-acting β₂-agonists are the drugs to be used in the therapy of asthma on aggravation and in the prevention of exercise induced bronchospasm and exhibit various side effects including sudden death whose cause is not clearly specified for asthmatic patients, such as the lowering of the potassium concentration in blood, the variation of blood pressure, the increase of heart rate, and the prolongation of Q-T interval and skeletal muscle tremor. It can be thought that these side effects can be caused particularly by too much use (Burgraff et al., Thorax, 56, 567-569, 2001, Bennet et al., Thorax, 49, 771-774, 1994, and Rave, Respi. Med., 95, 21-25, 2001).

### DISCLOSURE OF THE INVENTION

As described above, the development of drugs which further reduce the side effects of β₂-agonists including the increase of heart rate and the variation of blood pressure can be thought an extremely important clinical problem. Generally, short-acting β₂-agonists are frequently used as inhalations rather than by oral administration. With them, there are some side effects as described above here and there.

The present invention has an object to reduce the side effects of the drugs of the type, such as β₂-agonists, whose target site to bring about the effect is different from the site to express side effects.

As explained in the paragraph of Prior Art, the prior art drugs are all prodrugs that are converted to their active forms by utilizing the enzymatic activity accelerated in a cancer, an inflammatory tissue or the like. On the other hand, the present invention is a prodrug which utilizes differences in the enzymatic activities present among organs even in the normal state in the enzymatic activity present in a tissue or an organ.

The present inventors have made investigations on the glucuronic conjugate of 11-ethyl-7,9-dihydroxy-10,11-di-hydrobenzo[b,f]thiepin. This compound quickly undergoes glu-curonidation in the liver after oral administration and not less than 99% of it exist as the glucuronic conjugate in the blood. However, the compound has exhibited a pharmacological activity in the pharmacological target tissue of the lungs. As the result of the investigations, it has been found that the glucuronic conjugate of the compound undergoes the deglucuronidation in the lungs and it has been presumed that the parent compound formed by the deglucuronidation with β-glucuronidase has exhibited activity. (The details were described as Reference Example 1 following Examples.)

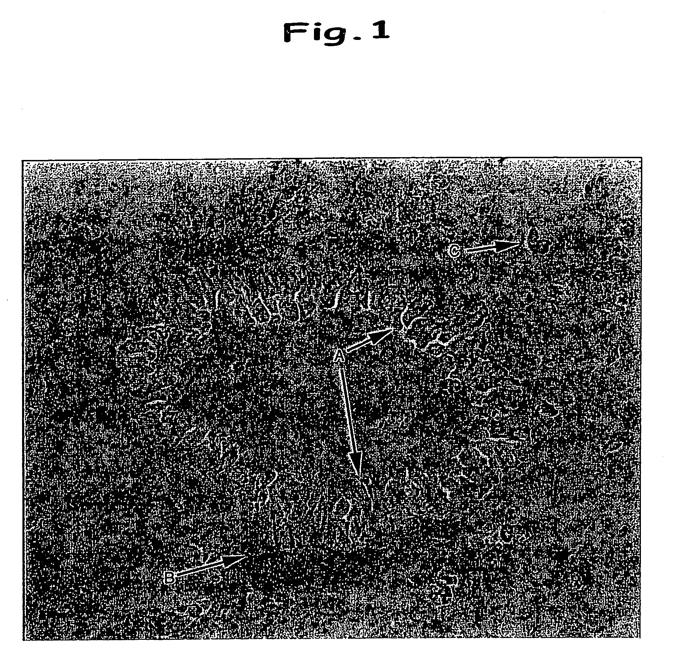

No detailed research report on the localization of β-glucuronidase in the level of each organ or a cell has been found. Then, when the localization of β-glucuronidase in the bronchi is confirmed, it has been found that β-glucuronidase is localized in the epithelium of the bronchioles as shown in Fig. 1 (the dark stained portions in Fig. 1 being β-glucuronidase). (The test method was described in detail as Reference Example 2 following Examples.) Thus, the present invention is based on the founding that the β-glucuronidase activity is localized in a specified site.

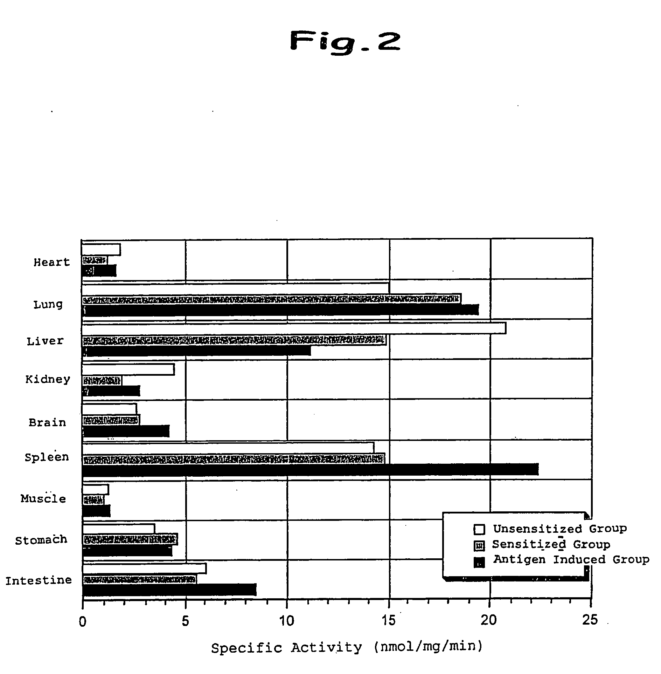

Furthermore, the examination was made on what organs in the body of living contain much β-glucuronidase. Parti-cularly, in consideration of the reduction of the side effects of β₂-agonists on the heat and the blood pressure, the examination of the β-glucuronidase activity in the lungs and the heart out of various organs was thought very important for making the glucuronidated prodrugs of β₂-agonists, and the β-glucuronidase activity in each organ of a guinea pig was measured. In this instance, with the supposition of an actual asthmatic state, the β-glucuronidase activity in an asthmatic state was compared by using an asthmatic animal model. In other words, the β-glucuronidase activities in each organ of a guinea pig sensitized with an antigen, a nonsensitized guinea pig, and a guinea pig sensitized with an antigen and having a stroke induced by the stimulus of the antigen were compared. The results are shown in Fig. 2. (The details of the test method were described as Reference Example 3 following Examples.)

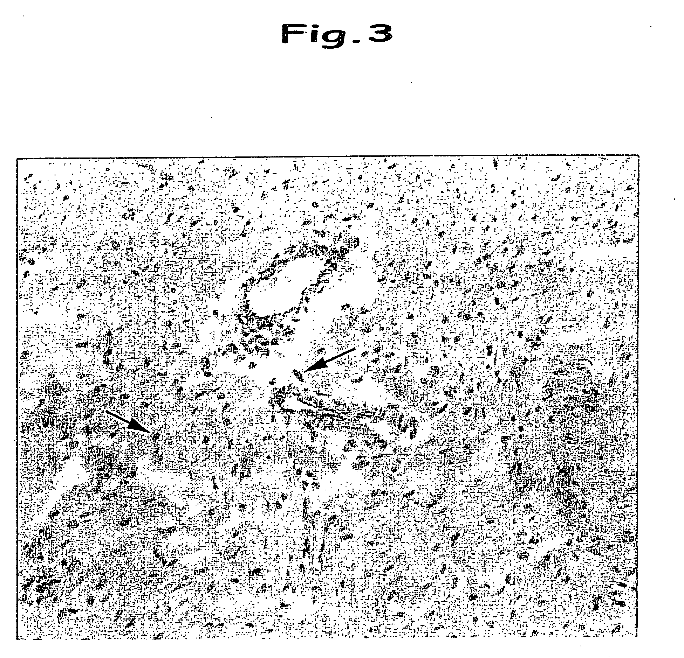

As would be clear from Fig. 2, it can be understood that the enzyme activity to be accelerated by the inflammatory state is very small and the amount of the enzyme activity present at the normal state is greatly different from that in each tissue. Further, it was confirmed that the enzymatic activity is low in the heart which is the site for β₂-agonists to express side effects. Additionally, the presence or absence of the localization of β-glucuronidase in the heart was confirmed, and no positive image showing the activity of β-glucuronidase was recognized at all as shown in Fig. 3 (the black portions indicated by arrows showing images of the cell nuclei stained with hematoxlin). (The details of the test method were described as Reference Example 4 following the Examples).

It is reported that the culture epithelium the constitutive liberation of β-glucuronidase into the culture medium (Scaggiante et al., Exp. Cell Res., 195, 1940198, 1991). In the same manner the culture human-lung macrophage effects the constitutive liberations of β-glucuronidase into the culture medium (Triggiani et al., The J. Immunol., 164, 4908-4915, 2000). Accordingly, in order for β-glucuronidase to express its extracellular activity, the liberation of β-glucuronidase by inflammation and cell damage is not necessarily required but at what high concentration β-glucuronidase is present in the part of the tissue is thought to be important. In cytohistological consideration from this viewpoint, it can be said that the neighborhood of the bronchiolar epithelium of the lungs is the site in which β-glucuronidase has a surprising local activity.

The present inventors has previously synthesized a compound of a glucuronidated β₂-agonist based on the above described knowledge and effected the inhalation of this glucuronic conjugate to allow it to undergo deglucuronidation with the β-glucuronidase which is abundantly present in the bronchioles of the lungs to express a bronchodilation effect at the local site and, in thinking that even if part of the glucuronic conjugate should reach the heart, hardly any specific side effect of β₂-agonists is recognized in the heart in which β-glucuronidase is hardly present, and actually synthesized the glucuronides of β₂-agonists to administer them by inhalation to a guinea pig model of the antigen induced type which is frequently employed and perfectly have shown an airway contractile inhibition and subsequently have clearly shown by using rats as the result of the investigations of the side effects on heart rate and blood pressure that the glucuronides of β₂-agonists have no side effect at all, and thus have completed the present invention.

The present invention provides a compound of the formula (1)

R-Drug (1)

(wherein R means a substituent cleavable with an enzyme whose activity is different in between the target site of Drug and the site to express side effects; and Drug is a drug to be activated by cleaving the substituent with the enzyme) or its physiologically acceptable salt.

Further, the present invention provides a compound of formula (2)

R'-Drug (2)

(wherein R' means a substituent cleavable with β-glucuronidase existing at a high activity in respiratory organs and at a low activity in the heart; and Drug means a drug for respiratory organs to be activated by cleaving the substituent with the enzyme) or its physiologically acceptable salt.

Furthermore, the present invention provides a drug composition comprising an effective amount of the compound of formula (1) or (2) together with pharmaceutically appropriate and physiologically acceptable fillers, additives and/or other active compounds and auxiliaries.

In addition, the present invention provides the use of the compound to be represented by formula (1) or (2) or its physiologically acceptable salt for preparing a pharmaceutical composition which is a prodrug utilizing an enzyme whose activity is different in between the target site of the Drug and the site to express side effects and expressing the effect of the Drug in the target site alone.

Still more, the present invention is to provide a method for preparing a β₂-agonist having a sugar as the substituent which comprises reacting a β₂-agonist having a hydroxyl group with a sugar halide derivative in any one of solvents of acetone, acetonitrile, dioxane, and tetrahydrofuran in the presence of a base and subjecting the resulting product to deprotection by alkali hydrolysis.

Still further, the present invention is to provide a method for preparing a β₂-agonist having a sugar as the substituent which comprises adding a benzyl halide derivative to a mixture containing a β₂-agonist having a plurality of hydroxyl groups and a base to selectively protect the hydroxyl groups, then conducting glycosylation, subjecting the intermediate to alkali hydrolysis, and then conducting hydrogenation.

The compound represented by formula (2) is a drug which is cleaved in the epithelium of the bronchioles of the lungs and whose active form directly acts on the bronchial smooth muscle to exhibit the drug effect when administered by inhalation and is a prodrug to be cleaved by the β-glucuronidase activity mainly derived from the epithelium not by the β-glucuronidase activity derived from the activation of cells by inflammation such as leukocytes.

Thus, the present invention is to provide a therapeutic method for respiratory disorders which comprises adminis-tering an effective amount of a compound of formula (2)

R'-Drug (2)

(wherein R' means a substituent cleavable with β-glucuronidase existing at a high activity in a respiratory organ and at a low activity in the heart; and Drug means a drug for respiratory disorders to be activated by cleaving the substituent with the enzyme) or its physiologically acceptable salt to a patient needing the therapy of respiratory disorders. In this therapeutic method, bronchodilators, β₂-agonists and the like can be illustrated as the drugs for respiratory organs and concretely, salbutamol, salmeterol, mabuterol, clenbuterol, pirbuterol, procaterol, fenoterol, tulobuterol, formoterol, hexoprenaline, terbutaline, trimetoquinol, chlorprenaline, orciprenaline, methoxyphenamine, methylephedrine, ephedrine, isoprenaline and the like can be mentioned.

A preferable embodiment of the present invention is a prodrug for respiratory organs comprising O-glucuronide of a β₂-agonist which has a hydroxyl group, in particular, a phenolic hydroxyl group. Examples of such a prodrug include 3-O-(β-D-glucuronyl)salbutamol, 3-O-(β-D-glucuronyl)salmeterol, 3-O-(β-D-glucuronyl)pirbuterol, 3-O-(β-D-glucuronyl)fenoterol, 3-O-(β-D-glucuronyl)tulobuterol, 4-O-(β-D-glucuronyl)formoterol, 3 or 4-O-(β-D-glucuronyl)hexoprenaline, 3-O-(β-D-glucuronyl)terbutaline, 6 or 7-O-(β-D-glucuronyl)trimetoquinol, 3-O-(β-D-glucuronyl) orciprenaline, 3 or 4-O-(β-D-glucuronyl)isoprenaline, and 8-O-(β-D-glucuronyl)procaterol.

In the prior art technology as explained in the paragraph of Prior Art, without a contrivance including the insertion of a spacer between a drug and glucuronic acid, the glucuronide of the drug could not efficiently be decomposed into the drug and glucuronic acid with β-glucuronidase in the tumor cell but the present invention has proved that without inserting such a spacer sequence, the drug of a glucuronide inhaled in the local site of the bronchioles of the lungs is efficiently decomposed.

The present invention does not utilizes the accelerated β-glucuronidase activity in the tumor tissue and inflammatory tissue but has been completed by finding the tissue having a very high β-glucuronidase activity in the normal state. Since there is a high β-glucuronidase activity in the epithelium of the bronchioles, the insertion of a spacer to effect efficient decomposition is thought not inevitably necessary but any appropriate spacer may be inserted for the sake of simplicity in the aspect of synthesizing a prodrug, safety and the like.
The compound obtained by inserting such a spacer between R and Drug in formula (1) or the compound obtained by inserting such a spacer between R' and Drug in formula (2) is included in the scope of the present invention.

### BRIEF DESCRIPTION OF THE DRAWINGS

Fig. 1 is a microscopic photograph showing that β-glucuronidase is strongly localized in the bronchiolar epithelium of the guinea pig lungs.
Fig. 2 is a graph showing the β-glucuronidase activity in each organ of guinea pigs.
Fig. 3 is a microscopic photograph showing no recogni-tion of the β-glucuronidase activity in the guinea pig heart.
Fig. 4 is a graph showing the inhibition of salbutamol glucuronide on the antigen inducing airway contraction reaction in guinea pigs.
Fig. 5 is a graph showing the inhibition of isoprenaline glucuronide on the antigen inducing airway contraction reaction in guinea pigs.
Fig. 6 is a graph showing the effect of salbutamol glucuronide on blood pressure and heart rate.
Fig. 7 is a graph showing the effect of isoprenaline glucuronide on blood pressure and heart rate.

### BEST MODE FOR CARRYING OUT THE INVENTION

The present invention is a prodrug utilizing a difference in the enzymatic activity in between the target site and the site to express side effects which is different from the one utilizing the enzymatic activity accelerated in the cancer cell and the inflammatory tissue or utilizing the enzymatic activity possessed by intestinal bacteria as shown in the prior art.

Accordingly, in the present invention, the drug has to be a drug whose target site is different from the site to express side effects. Particularly, the drug whose site to express side effects is specified and restricted is preferred. Various receptor agonists and blockers having restricted sites in which the receptors of the target site and the site to express side effects exist are present can be the drugs of the present invention.
Further, from the necessity that the drug of the present invention is bonded to a substituent cleavable with an enzyme, the drug having suitable chemical structure for the bonding is preferred. For example, in the case of bonding a sugar cleavable with β-glucuronidase to the drug, drugs having a hydroxyl group, an amino group, a carboxyl group and/or a thiol group are preferred. Especially the drug having a chemical structure containing a hydroxyl group, especially, a phenolic hydroxyl group is suitable from the point of the stability of the compound and easiness of cleavage by glucuronidase. Many of β₂-agonists have a chemical structure containing a hydroxyl group, especially, a phenolic hydroxyl group and thus are suitable for the drugs of the present invention.

The target site means cells, tissues, organs and the like in which the drug exhibits the drug effect. Further, the site to express side effects means cells, tissues, organs and the like in which the drug exhibits unfavorable effects.

In the present invention, the respiratory organ means the airway and the lungs.

When the target site is the respiratory organ, the drugs of the present invention are therapeutic drugs for disorders such as bronchial asthma, infantile asthma, chronic bronchitis, acute bronchitis, pneumonia, pulmonary emphysema, and pulmonary tuberculosis,

When the site to express side effects is the heart, the drugs of the present invention are those including, for examples, β₂-agonists which target other organs than the heart and express side effects on the heart.

In the present invention, the bronchodilator means a drug which directly or indirectly acts on the bronchial smooth muscle by inhalation or the like. In the bronchi, β-glucuronidase is localized in the epithelium of bronchioles and the like, and when the drug is liberated there, it can be allowed to effectively act on the smooth muscle immediately under the epithelium.

As the β₂-agonists in the present invention, salbutamol, salmeterol, mabuterol, clenbuterol, pirbuterol, procaterol, fenoterol, tulobuterol, formoterol, hexoprenaline, terbuta-line, trimetoquinol, chlorprenaline, orciprenaline, methoxy-phenamine, methylephedrine, ephedrine, isoprenaline and the like can be illustrated as the representatives, and not only their derivatives but also any drug having β₂-action may be used.

As the enzymes of the present invention, glycosidases such as β-glucuronidase, glucosidase, galactosidase, N-acetyl-D-glucosaminidase, N-acetyl-D-galactosaminidase, man-nosidase, and fucosidase, and arylsulfatase can be illus-trated. In the case of the agents for respiratory organs, β-glucuronidase is particularly preferred.

When the enzymes are the above described glycosidases, sugars to be selected from D-glucuronic acid, D-glucose, D-galactose, N-acetyl-D-glucosamine, N-acetyl-D-galactosamine, D-mannose, and L-fucose and the like can be illustrated as the monosaccharide in the present invention, and oligosaccha-rides consist of two to five of the above described mono-saccharides which are bonded to each other through an α- or β-O-glycosidic bond can be illustrated. Normally, the bond between the monosaccharide and the drug is an α- or β-O-glycosidic bond. When the enzyme is β-glucuronidase, a β-glucuronyl bond is preferred.

The substituent in the present invention means a sugar residue cleavable with an enzyme, a sulfate group and the like. For example, when the enzymes are glycosidases, glucu-ronyl, glucopyranosyl, galactopyransyl, acetyl-glucosamyl, acetyl-galactopyranosyl, acetylpyranosyl, mannopyranosyl, fucopyranosyl and the like can be illustrated as the sugar residues.

Without directly bonding a drug to the substituent, it is possible to bond the drug to β-gluruconide as the trigger through a spacer as shown in J. Med. Chem., 2000, 43, 475. The spacer is a structure to be placed between a drug and a substituent. The spacer is preferably the one that is chemically or enzymatically cleaved in the target organ to quickly express the parent compound. In this instance, it is desired that the spacer is nonselectively cleavable, and the one decomposable simply by hydrolysis and the like is used.

In the present invention, a target organ having a high enzymatic activity is selected, and accordingly the drug is fully liberated in the target tissue without using any spacer as shown in the Examples. Thus, the spacer is not necessarily required but depending on the drug, the organ and the enzyme to be selected, it is sometimes advantageous to use a spacer.

When the cleavage with an enzyme is made easy through a spacer or the substituent has a low reactivity due to its steric hindrance, the effect of the spacer for easy conversion into the parent compound and the like can be thought. However, when a spacer is used, it becomes necessary that the pharmacological properties such as the toxicity of the spacer and its metabolite are clarified beforehand.

As the spacers, those which are chemically stable like an ester and carbamoyl and finally decomposed by an enzyme to quickly express the parent compound are widely used for a long time (H. Bundgaard Ed., Design of Prodrugs, p.262-269, 1985, Elsevier). Depending on the drugs, those disclosed in U.S. patent No. 5,621,002 [Family Patents: European Patent Application Publication No. 642799 and Japanese Patent Publication (Kokai) No. Hei 7-149667/1995], U.S. Patent No. 5,935,995 [Family Patent: European Patent Application Publication No. 795334 and Japanese Patent Publication (Kbkai) No. Hei 10-1495/1998], and U.S. Patent No. 5,955,100 [Family Patent: European Patent No. 5935995, Japanese Patent Publication (Kokai) No. Hei 6-293665/1994] and the like can also be used.

As the bonding position of a sugar or a spacer, a phenol group, an imino group or an amino group can be thought in the example of β-agonists. A sugar portion or a sulfate group is directly or indirectly bonded to these substituents as the marks to form a prodrug.

As the concrete examples of the compounds of the present invention, 3-O-(β-D-glucuronic acid)-salbutamol, 4-O-(β-D-glucuronyl)-isoprenaline, iso-prenaline-4-O-sulfate and the like can be illustrated. The method for the preparation of the former two compounds was in the Examples. The last compound can be obtained by the reaction of isoprenaline with a complex of sulfur trioxide with trimethylamine.

It is preferred that the prodrug of the present invention is used by local administration. The possibility that other sites than the target site and the site to express side effects are susceptible to the enzymatic activity is decreased by local administration, and thus the prodrug of the present invention comes to a more effective prodrug. In the case of the agents for respiratory organs, the prodrug is preferably used as the pharmaceutical composition for inhalation.

When the prodrug is used as inhalations, any additive that is generally used in the pharmaceutical composition for inhalation may be used as the inhalation additive, and for example, propellants, solid fillers, liquid fillers, binders, lubricants, correctives, preservatives, stabilizing agents, suspending agents, dispersing agents, solvents, tonicity adjusting agents, pH adjustors, solubilizing agents and the like are used. As the propellants, liquefied gas propellants, compressed gas propellants and the like can be used. Further, the pharmaceutical composition of the present invention may contain, as the active component, a drug component in addition to the prodrug of the present invention.

In the pharmaceutical composition of the present invention, the content of the prodrug may vary depending on the drug, the target disorder, the age and the sex of the subject patient, the state of the disorder and the like and is about 0.01 to 99.9% by weight, preferably about 0.1 to 50% by weight, more preferably about 0.5 to 20% by weight based on the entire pharmaceutical composition. The content of various additives such as inhalation additives may vary depending on the target disorder, the age and sex of the subject patient, the state of the disorder and the like and is about 0.1 to 99% by weight, preferably about 10 to 99% by weight, more preferably about 50 to around 99% by weight, particularly preferably about 70 to around 99% by weight.

When the pharmaceutical composition of the present invention is used as inhalations, it can be made into powdered inhalations, inhalation suspensions, inhalation solutions or encapsulated inhalations by using the conventional and can be applied by an appropriated inhaler in use, and particularly powdered inhalations are preferably used. Furthermore, the pharmaceutical composition of the present invention can be used as aerosols.

When the pharmaceutical composition of the present invention is used, commercially available inhalers may be used as the appliances to be used in its application. For example, VENTOLIN ROTACAPS (Glaxo), SPENHALER (trademark, Fujisawa Pharmaceutical Co., Ltd.), INTAL SPINCAPS (Fisons), ATROVENT AND BEROTEC INHALETTEN (Boehringer Ingelheim), FORADIL (Ciba), BENTODISKS (Glaxo), Pavlyzer (trademark, Teijin Ltd.), BRICANYL TURBUHALER (Astra), MIAT INSUFFLATOR and the like can be illustrated.

The prodrug of the present invention is only bonded to a sugar and the like as the substituent which are normally safely metabolized in the body, and thus the possibility that its toxicity becomes higher than the toxicity of the drug as such is low. The prodrug is suited in use for local administration, and thus can be spared with a minimum effective dose and a generalized large dose can be avoided. Accordingly, even children can be easily and safely dosed. Particularly when the prodrug is made into inhalations and aerosols, remarkable local actions and effects can be exhibited.

Needless to say, administration of the prodrug of the present invention such as intravenous administration and intramuscular administration causes much more enhancement of safety than administration of a drug per se without using the prodrug thereof. For example, in case of emergency of asthmatic attack, intravenous administration of a β₂-agonist may be necessary. In such a case, since glucuronidase activity is lower in the heart, side effects of a β₂-agonist in the heart are decreased more remarkably by administration of the prodrug than by administration of the drug per se.

The dose of the pharmaceutical composition of the present invention may vary depending on the drug, the target disorder, the age, the weight, the state of disorder, the route of administration, the number of administration and the like and, for example, in the case of β₂-agonists, nearly the same effect as with the dose of an active drug before making its prodrug can be exhibited.

As the method for preparing the prodrug of the present invention, there are organic chemistry-based glycosylation and enzymatic glycosylation. For example, a sugar derivative whose hydroxyl groups have been protected is subjected to the glycosidation reaction represented by the Koenigs-Knorr reaction (Advances in Carbohydrate Chem. and Biochem., 57, 207, 2001, Academic Press) to form a desired glycosidic bond, and then deprotection is conducted to obtain a target prodrug.

By the enzymatic method (KISO TO RINSHOU, 30, 2403, 1996) as well, the same result can be obtained by combining glycosyltransferase with an UDP-sugar derivative.

### EXAMPLES

The present invention will be explained in more detail by examples. The present invention is by no means limited by these examples.

### [Example 1]

### Preparation of Salbutamol Glucuronide [3-O-(β-D-Glucuronyl)-salbutamol] (Compound of Example 1)

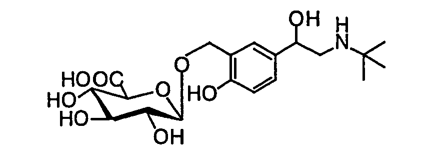

### (1) Preparation of 2-(4-methoxybenzyloxy)-5-(2-(N-tert-butylamino)-1-(4-methoxybenzyloxy)ethyl)benzyl alcohol

To a mixture of 1.466 g of salbutamol, 25 mg of NaI, and 5 mL of tetrahydrofran (THF), 250 mg of NaH was added little by little at -78°C. The resulting mixture was stirred at 0°C for 15 minutes, then added with 1.125 g of p-methoxybenzyl chloride (p-MeO-benzylchloride) at -78°C, and subsequently stirred at room temperature for 16 hours. The reaction mixture was added with acetone and filtered, and the filtrate was concentrated, and then subjected to column chromatography to obtain 1.20 g (59 %) of a target substance.
nmr (CDCl₃): 1.19 (9H, s), 2.60 (2H, m), 2.62 (2H, m), 3.53 (1H, d, J=10 Hz), 3.81 (6H, s), 3.88 (1H, d, J=10 Hz), 3.93 (1H, m), 4.60-4.70 (2H, m), 4.99 (1H, s), 7.2-7.8 (11H, m)

### (2) Glycosidation and Deprotection

To 4 mL of dichloromethane were added 1.04 g of 2-(4-methoxybenzyloxy)-5-(2-(N-tert-butylamino)-1-(4-methoxybenzyloxy)ethyl)benzyl alcohol, 1.50 g of acetobromoglucuronic aid methyl ester (bromo-2,3,4-tri-O-acetyl-β-glucopyranuronic acid methyl ester), 1.577 g of silver carbonate, and 1.57 g of MS4A and stirred overnight at room temperature. The reaction solution was filtered with Celite and the filtrate was concentrated, and then separated by column chromatography to obtain 1.68 g (82%) of a crude product. This product was dissolved with methanol (MeOH)-THF (5 mL, a 2:3 mixture), added with 20% NaOH (2.59 mL) and stirred at room temperature for one hour. The reaction was confirmed by thin-layer chromatography (TLC) [at a ratio of ethyl acetate (AcOEt) to n-hexane (n-Hex) of 1/2), and the reaction product was neutralized with acetic acid under cooling with ice. The resulting product was added with Pd-C (100 mg) and hydrogenerated (at room temperature, overnight). The reaction solution was filtered, and then the filtrate was concentrated and subjected to LH-20 column chromatography to separate 138 mg (12%) of a target substance (Compound of Example 1).
nmr (DMSO-d₆): 1.25 (9H, s), 2.80-2.94 (2H, m), 3.05-3.35 (4H, m), 4.25 (1H, m), 4.60 (1H, m), 4.76 (1H, d, J=10 Hz), 4.78 (1H, d, J=8.8 Hz), 6.80 (1H, d, J=8.2 Hz), 7.12 (1H, dd, J=8.2 Hz & 1.6 Hz), 7.44 (1H,d, J=1.6 Hz)
IR (KBr, cm⁻¹): 3402, 2980, 1617, 1509, 1406, 1276, 1200, 1118, 1074

### [Example 2]

### Preparation of Isoprenaline Glucuronide [4-O-(β-D-Glucuronyl)-isoprenaline] (Compound of Example 2)

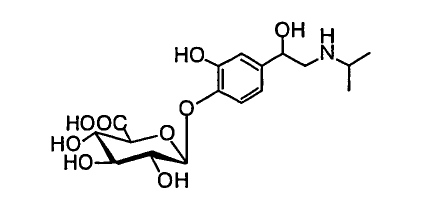

To a mixture of 1.00 g of isoprenaline hydrochloride and 1N-NaOH (4.00 mL), 4.16 mL of acetone dissolving 1.28 g of bromo-2,3,4-tri-O-acetyl-β-D-glucopyranuroic acid methyl ester was added little by little at 0°C and left to stand at room temperature. The reaction was conducted for two days at room temperature while maintaining the pH in the neighbor-hood of 7 with the addition of 1N-NaOH from time to time. The reaction solution was concentrated, then added with 20% NaOH (2 mL) and stirred at room temperature for 30 minutes. The resulting solution was cooled, and then attentively added with acetic acid to render the pH 2 to 3, and separated by a HP-20 column, and subsequently further separated with a LH-20 column to obtain Compound of Example 2. The yield was 81 mg (5.2%).
nmr (DMSO-d₆): 1.22 (6H, d, J=6.5 Hz), 2.82-2.96 (2H, m), 3.05-3.35 (4H, m), 3.30 (1H, m), 4.70 (1H, brs), 4.70 (1H, m), 6.80-7.50 (3H, m)
IR (KBr, cm⁻¹): 3402, 1617, 1509, 1400, 1287, 1068

### [Example 3]

### Preparation of 3-(β-D-Glucuronyloxy)methyl-4-hydroxy-α-{[(4-methoxy-α-methylphenethyl)amino]methyl} benzyl alcohol

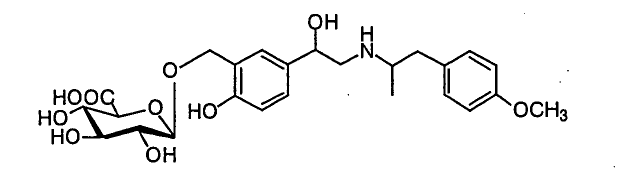

The titled compound was obtained by the same process as in Example 1.
nmr (DMSO-d₆): 0.90 (3H, d, J=6.21Hz), 2.82-2.96 (2H, m), 3.05-3.35 (4H, m), 3.30 (1H, m), 3.71 (3H, s), 4.45 (1H, brs), 4.47 (1H, m), 6.60-7.30 (7H, m)

### [Example 4]

### <<Pharmacological Activity of β₂-Agonist Glucuronide>>

### 1. Test method

In order to examine the bronchodilation effect of the glucuronides of salbutamol and isoprenaline of β₂-agonists, an asthmatic model of a guinea pig sensitized with ovalumin was used. The test was conducted in accordance with the method of Konzett and Rossler (Arch. Exp. Pathol. Pharmakol., 195, 71-75, 1940).

### <Sensitization>

On day 1 and day 8 after the initiation of sensitization, 500 µg/0.5 mL of ovalbumin was intramuscularly administered to both legs of a guinea pig and 1.5×10⁵ cells/mL/animal of pertussis vaccine was intraperitoneally administered to effect active sensitization. On day 15 after the initiation of sensitization, 10 and 100 µg/site of ovalbumin were intradermally administered to the back of the guinea pig, respectively, to check the state of sensitization. Only the animals which were found positive by the sensitization check 6 hours after the intradermal sensitization were used in the experiment.

### <Method of Administration>

As the test substance, a drug solution was atomized by reducing the atomizing amount of an ultrasonic nebulizer to generate an aerosol, and the aerosol was then led into an exposure chamber (M.I.P.S. Co.), sucked at a rate of 3 L/min with the use of an air pump (SPP-3GA, Techno Takatsuki Co.) and administered to the guinea pig for 10 minutes by inhalation 40 minutes before the challenge of ovalbumin.

### <Measurement of Airway Pressure>

On day 10 to 23 after the initiation of sensitization, the animals were anesthetized with sodium pentobarbital (50 mg/kg, i.p.), and cannulation was conducted into the trachea. Through the tracheal cannula the animals were connected to an artificial respirator and the change in the ventilation pressure under artificial respiration (a ventilation amount of 10 mL/kg, a ventilation frequency of 50 times/min) was recorded as the airway pressure through a differential pressure transducer (Validyne, Gould Electronics) connected to the tracheal cannula on a recorder (WT-645G, Nihon Koden Co., Ltd.). The airway pressure was measured up to 10 minutes after the administration of ovalbumin. Then, cannulation was conducted into the right and left common carotid veins. From the left cannulation, gallamine (10 mg/mL) in a volume of 1 mL/kg was intravenously administered (300 µg/kg) to confirm the disappearance of spontaneous respiration. Thereafter, ovalbumin was intravenously administered to induce an antigen-antibody reaction. The measuring points of the airway pressure were set at a time before induction, and at 1, 3, 5, 7, and 10 minutes after induction. The ratio of the increase in the airway pressure is represented by the percentage of the values obtained by subtracting the observed value before induction from the observed value at each measuring time after induction based on the maximum occlusion at each measuring time.

### <Test Materials>

As the test substances, salbutamol glucuronide and isoprenaline glucuronide were used. The salbutamol glucuronide is a white powder and the isoprenaline glucuronide is a brown crystal and both were stored at -80°C under protection from light. As the control substances for comparison, each of sulbutamol chloride (hereinafter referred to as sulbutamol) and isoprenaline chloride (hereinafter referred to as isoprenaline) was used. The salbutamol and the isoprenaline are white powders and were stored at room temperature under protection from light. The test substances and the control substances for comparison were weighed in a necessary amount and dissolved in physiological saline (products of Otsuka Pharmaceutical Factory, Inc., Lot Nos. 1D78 and 1E84) to effect the preparation before using.
The concentrations of the test substances and the control substances for comparison in solutions were rendered equivalent amounts by molar concentration. All solutions were nearly stable at room temperature for 24 hours.

Further, the salbutamol glucuronide and the isoprenaline glucuronide were used within 30 minutes after the prepartion. In addition, ovalbumin (OVA, a product of Sigma Chemical Company, Lot No. 120K7001), gallamine (gallamine triethiodide, Sigma Chemical Company, Lot No. 76H1106), sodium pentobarbital (Tokyo Chemical Company, Lot No. GI01), pertussis vaccine (Wako Pure Chemical Industries, Ltd., Lot No. SEK7880), and physiological saline (Otsuka Pharmaceutical Factory, Inc., Lot Nos. 1D78 and 1E84) were used.

The constitution of each test group is shown in Table 1.

**Table 1**

| Test Group | Route of Administration | Concentration of Drug (%) | Inhalation Time | Number of Animals Used |
|---|---|---|---|---|
| Control | Inhalation | 0 | 10 min | 8 |
| Salbutamol | Inhalation | 0.05 | 10 min | 8 |
| Salbutamol glucuronide | Inhalation | 0.072 | 10 min | 8 |
| Isoprenaline | Inhalation | 0.1 | 10 min | 8 |
| Isoprenaline glucuronide | Inhalation | 0.157 | 10 min | 8 |

### <Statistical Analysis Treatment Method>

The obtained experimental results were shown by mean values and standard deviations with airway pressure. As to the test of significance, the Student's t test without any correspondence was conducted when two groups were compared. When multiple groups were compared, the Dunnett's multiple test was conducted. In either method the significant standard was regarded as 5%. The inhibition ratio of each test substance against the increase of the airway resistance was calculated as the inhibition ratio against the control group when the inhibition ratio of the control group was regarded as 0%.

### 2. Result

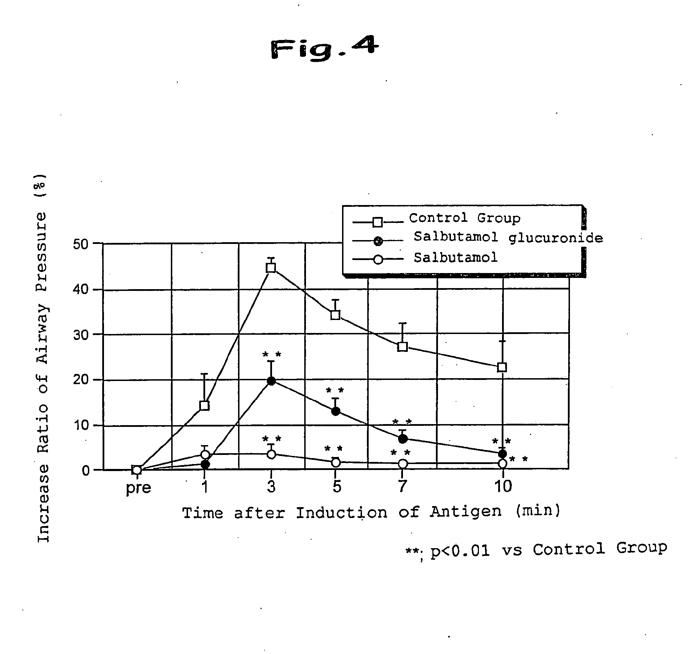

The results of examining the effects of salbutamol and salbutamol glucuronide on the antigen inducing immediate type asthmatic reaction are shown in Fig. 4.
The results were shown by an increase ratio based on the airway pressure before the administration (pre) of the antigen of ovalbumin as described in the method. The term "pre" in the Figure indicates the point of time when gallamin was administered and spontaneous tension was allowed to disappear to stabilize the airway and meant about 5 to 10 minutes before the administration of ovalbumin was initiated. With the control group of guinea pigs in which the antigen-antibody reaction was induced by the intravenous administration of ovalbumin, the airway pressure quickly increased in one minute after the antigen induction and showed a maximum increase of about 44% in three minutes. With the group of guinea pigs allowed to inhale salbutamol at a concentration of 0.05%, an increase in the airway pressure of about 3% was shown in three minutes after the antigen induction to strongly inhibit the increase of the airway pressure. This result showed a significant inhibition of about 92% compared to the control group. On the other hand, the group allowed to inhale salbutamol glucuronide at a concentration of 0.072% as well showed an increase ratio of about 20% after three minutes to strongly inhibit the increase of the airway pressure. This result showed a significant inhibition of about 56% compared to the control group.

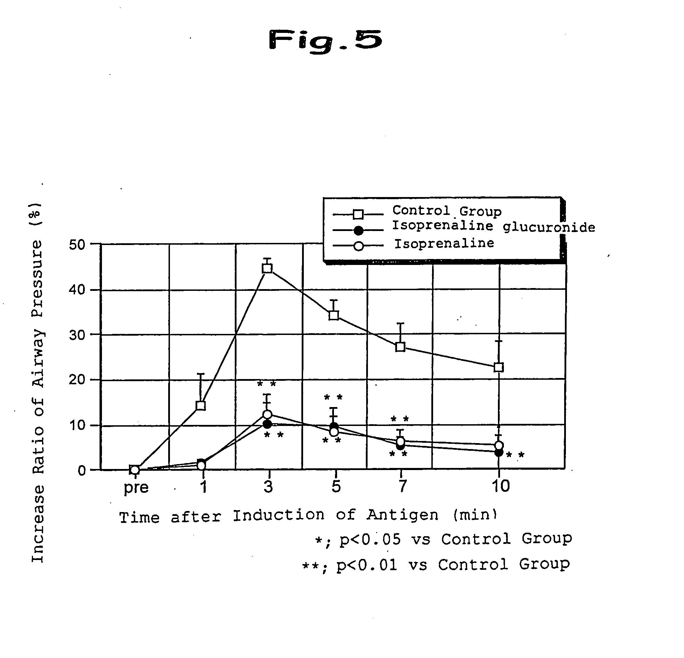

On the other hand, the results of examining the effects of isoprenaline and isoprenaline glucuronide on the antigen inducing immediate type asthmatic reaction is shown in Fig. 5. The experimental conditions and the presentation of the results are the same as in the case of sabutamol. The group allowed to inhale isoprenaline at a concentration of 0.1% showed an increase in the airway pressure of about 12% three minutes after the antigen induction to strongly inhibit the increase of the airway pressure. This result showed a significant inhibition of about 73% compared to the control group. On the other hand, the group allowed to inhale isopregnaline glucuronide at a concentration of 0.157% as well showed an increase ratio of about 10% after three minutes to strongly inhibit the increase of the airway pressure.
This result showed a significant inhibition of about 77% compared to the control group.

From the above it has been clarified that salbutamol glucuronide and isoprenaline glucuronide can significantly inhibit the immediate type asthmatic reaction of guinea pigs by inhalation administration. From this result and the result (Fig. 1) that the β-glucuronidase activity is strongly present in the bronchiolar epithelium, it can be thought that salbutamol glucuronide and isoprenaline glucuronide enter the trachea by inhalation administration and are hydrolyzed with the β-glucuronidase in the neighborhood of the bronchioles of the lungs and converted to the active forms of salbutamol and isoprenaline to exhibit an antiasthmatic effect.

### [Example 5]

### <<Influence of β₂-Agonist Glucuronide on Side Effects>>

By using β₂-agonists of isoprenaline and salbutamol, the influence of these β₂-agonists and their glucuronides (Example 1 and Example 2) on the heart was confirmed.

### 1. Test Method

The test was conducted by using Crj:CD(SD) rats with each group of 6 rats.

### <Measurement of Blood Pressure and Heart Rate>

Surgery was carried out under the anesthesia of a mixed gas of oxygen, nitrous oxide and isoflurane, and one end of a polyethylene tube (PE50, Becton Dickinson) filled with physiological saline containing heparin (100 U/mL) was inserted into a common carotid artery and indwelled, and the other end was passed through the neighborhood of the neck of the back of the animal and connected to a cannula (Instec Co.) installed at the upper portion of an exposure chamber. This cannula was connected to a pressure transducer (P23XL. Gould Electronics) through a polyethylene tube. Further, the periphery of the polyethylene tube from the back of the rat up to this cannula was passed through a metallic spring to prevent a damage to be caused by the rat.

The administration of the drugs was conducted by preparing an exposure chamber and subjecting the rat with a cannula for measuring blood pressure in the indwelled state in the common cartoid artery to the generaliged inhalation exposure. This method is typically used in the generalized inhalation exposure.

The signals from the pressure transducer (P23XL, Gould Electronics) were led to a pressure processor signal conditioner (Gould Electronics) and recorded on a thermal array recorder (RS3400, Gould Electronics). The blood pressure and the heart rate were continuously recorded from before the initiation of administration to 20 minutes after the completion of administration. The administration of the drugs was initiated when not less than one hour after waking passed and the measurement parameters were stabilized.

### (Test Materials)

Test substances were prepared in the same manner as in Example 1.

The constitution of each test group is shown in Table 2.

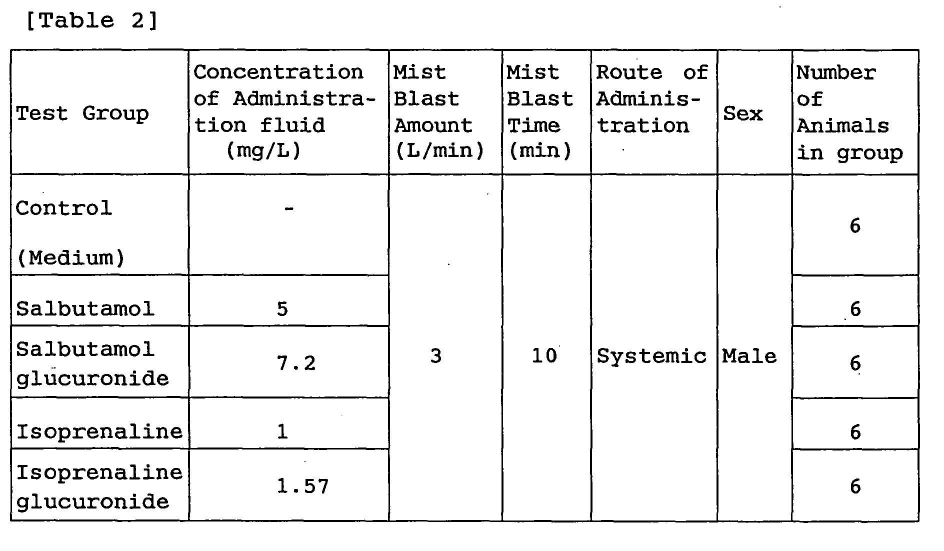

### <Statistical Analysis Treatment Method>

The representative values of each group were shown by mean values [standard deviation (S.E.)]. With the mean values of each group, the test of significance was conducted by the Tukey's multiple test. Further, the significant standard was regarded as 5%.

### 2. Result

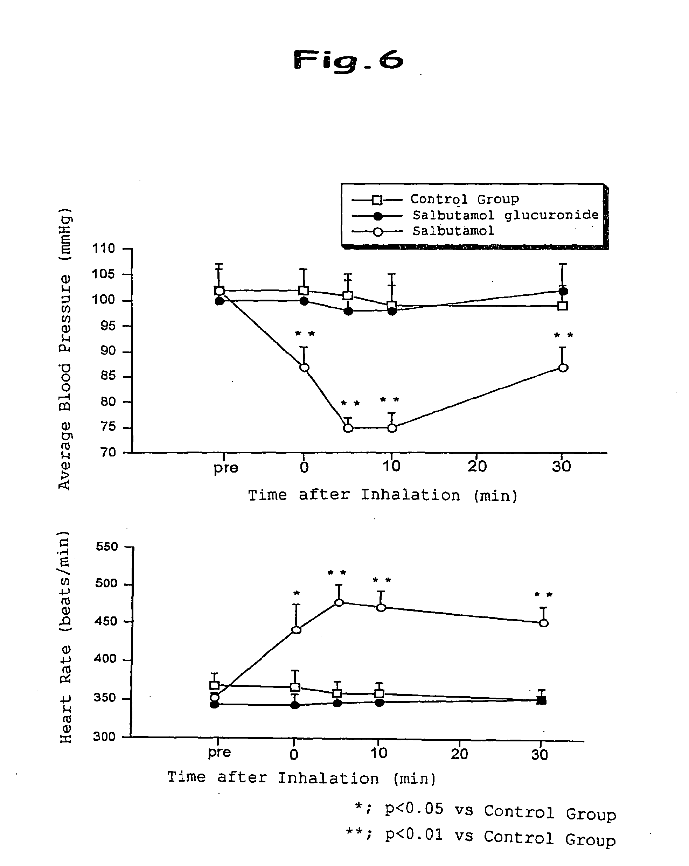

The results of examining the effects of salbutamol and salbutamol glucuronide on the heart function, particularly the blood pressure/heart rate is shown in Fig. 6. Further, the term "pre" in the Figure means immediately before initiating the administration of the drugs by inhalation. Immediately after administering salbutmol by inhalation, the decrease of the blood pressure and the increase of the heart rate were recognized. Five minutes after the completion of inhalation, the decrease of the blood pressure to 75 mmHg was shown, and this decrease was a decrease of about 26% of the blood pressure before inhalation (pre). Further, with the heart rate, the same tendency was recognized, and after five minutes an increase of a maximum of about 36% was shown. The maximum blood pressure decrease ratio and the maximum heart rate increase ratio during 30 minutes of the measurement with time were 27% and 39%, respectively. Thus, it has become clear that the inhalation of salbutamol remarkably affects the blood pressure and the heart rate. Contrast to this, as the result of the same test conducted by using salbutamol glucuronide, no effect was observed at all as in the control group (inhalation of physiological saline).

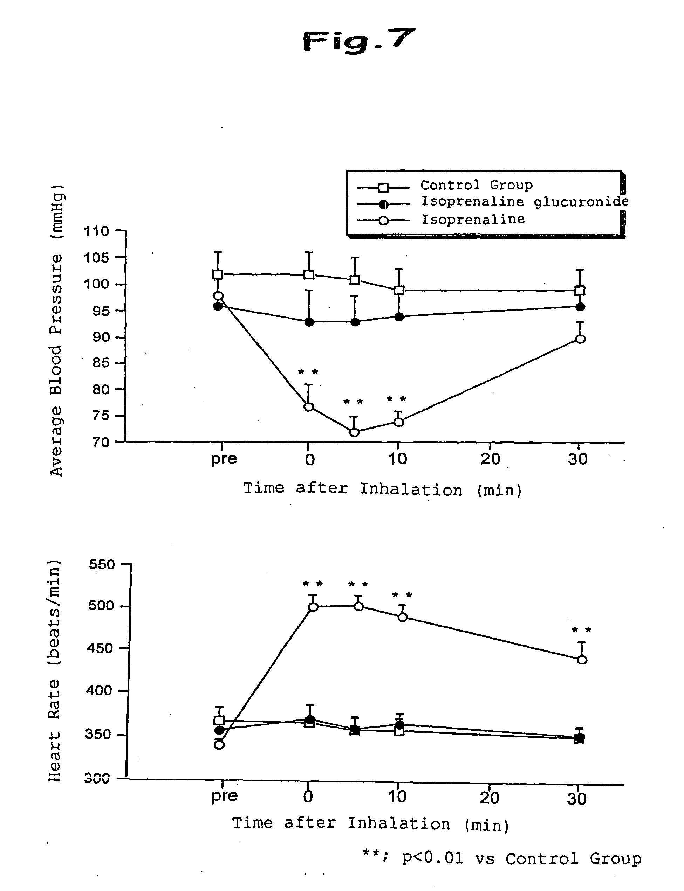

On the other hand, with isoprenaline, immediately after the administration by inhalation, the decrease of the blood pressure and the increase of the heart rate were recognized as in the case of salbutamol, and five minutes after the completion of inhalation, the decrease of the blood pressure to 72 mmHg was shown and this decrease was a decrease of about 27% before the treatment. Further, with the heart rate, the same tendency was recognized, and after five minutes an increase of the heart rate of about 49% was shown (Fig. 7). The maximum decrease of the blood pressure and the maximum increase of the heart rate during 30 minutes of the measurement with time were 28% and 50%, respectively. Thus, it has become clear that the inhalation of isoprenaline remarkably affects the blood pressure and the heart rate. Contrast to this, as the result of conducting the same test with the use of isoprenaline glucuronide, no effect was recognized at all as in the case of the control group. It is known that especially isoprenaline has β₁-action and strong side effects on the heart. It has been shown that the present invention enables complete removal of the effect which a β₂-agonist possesses on the heart by using the β₂-agonist glucuronide. These results have elucidated that the glucuronides of salbutamol and isoprenaline allow the serious side effects of salbutamol and isopregnaline on the blood pressure/heart rate to disappear.

### [Reference Example 1]

The present invention was explained by using salbutamol and isoprenaline which belong to β-agonists as examples but this concept presents an example that not only these two compounds as shown in the Examples but also other compounds having entirely different structures therefrom exhibit the same tendency, that is, glucuronidated drugs can be hydrolyzed in the target tissue to express the activity of the drugs.

11-Ethyl-7,9-dihydroxy-10,11-dihydrobenzo[b,f]thiepin is a compound which shows an effect in the in vitro test for the inhibition of contraction with the use of a smooth muscle. The smooth muscle contraction induced in the tracheal smooth muscle sample of a pig with a high concentration of KCl or carbachol is inhibited with an IC₅₀ of about 5 µM. In orally administering this compound, the immediate asthmatic model (in the same experimental system as in Example 3) showed the airway contraction inhibition with 10 mg/kg. Thereafter, the investigations relating to the metabolism of the present compound were initiated, and it was found that this compound was adsorbed after oral administration and quickly subjected to glucuronidation.
In mice, rats, guinea pigs, dogs, and monkeys, as the result of blood analysis after the administration, not less than 99% of the compound was the glucuronic conjugate in any of the animal species. From this result, its O-glucuronide was synthesized in consideration of the possibility that the activity would exist in the conjugate. The synthesized O-glucuronide showed no effect in the in vitro contraction inhibition test using the smooth muscle as described above. On the other hand, when the effect of the O-glucuronide was confirmed by using the sensitized model of a guinea pig by intravenous administra- tion as described above, the inhibition of airway contraction could be confirmed. This can be assumed due to the result that the intravenously administered O-glucuronide reached the lung tissues and then was hydrolyzed to return to its unchanged original form to exhibit the inhibition of airway contraction.

Thus, even with the compound having a completely different structure from β-agonists, the recognition of the same result as in the case of the β-agonists strongly shows the possibility that the glucuronidated compound is efficiently hydrolyzed in the lungs to express the activity.

Now the Figs. 1, 2, and 3 explained in the paragraph "Means to Solve the Problems" will be explained in more detail.

### [Reference Example 2]

### <<Fig. 1: Localization of β-Glucuronidase in Lungs>>

The localization of β-glucuronidase in the lungs and the heart was analyzed with the use of an enzymatic histochemical technique.

### 1. Test Method

A tissue specimen of a guinea pig lung was prepared, and the vital staining was conducted with the use of naphthol AS-BI β-glucuronide substrate for β-glucuronidase according to the method of Fishman et al. (J. Hist. Cytochem., 12, 298-305, 1964). The removed lung was fixed with a 4% paraformaldehyde solution to prepare a 4 to 6 µm frozen section by a cryostat. The substrate solution was prepared by adding and dissolving 28 mg of naphthol AS-BI β-glucuronide in 1.2 mL of 0.05M sodium bicarbonate and adding a 0.2N acetic acid/sodium acetate buffer (pH 5) to the resulting solution up to 100 mL. The staining solution was prepared by adding 0.3 mL of a pararosaniline fluid to 0.3 mL of a 4% sodium nitrite solution to effect diazotization, adding 10 mL of the subs-trate solution to the resulting solution to adjust the pH to 5.2, then adding distilled water to the obtained solution up to 20 mL, and finally filtering the resulting solution with a filter paper. The staining solution was placed on the section to effect reaction at 37°C for two hours. After the reaction, the section was washed, dehydrated, and sealed according the conventional method.

### 2. Result

The result of preparing a frozen section and subjecting the tissue to vital staining by the β-glucuronidase activity is shown in Fig. 1. As shown in Fig. 1, strong positive images were recognized at the bronchiolar epithelium of the lung (regions shown by A in the Figure) and the alveolar macrophages (C in the Figure) (a portion to be seen as stained in black shows the activity of β-glucuronidase).

It is reported that the β-glucuronidase of the alveolar macrophages is high (Hayashi, J. Histochem. Cytochem., 15, 83-92, 1967 and Barry and Robinson, Histochem. J., 1, 505-515, 1969) but there has been no report that β-glucuronidase is localized in the epithelium constituting bronchioles. It has been shown that β-glucuronidase is particularly strongly expressed in the regions having contact with the outer part of the bronchioles of the lungs.

The possibility that a group of cells which most greatly participates in the β-glucuronidase activity of the lungs is not the cells of the inflammatory system but the bronchiolar epithelium has been suggested.

### [Reference Example 3]

### <<Fig. 2: Examination Relating to β-Glucuronidase Activity in Each Organ>>

In order to investigate the level of the β-glucuronidase activity in each organ and the variation of this enzymatic activity in an asthmatic model animal, a sensitized guinea pig as established as the immediate asthmatic model was prepared, and the enzymatic activity of each organ was compared with that of the unsensitized guinea pig. Further, with the sensitized guinea pig, the enzymatic activity in an antigen induced individual was compared with that in an antigen uninduced individual.

### 1. Test method

### <Sensitization>

The sensitization was actively conducted by intramuscularly administering 500 µg/0.5 mL of ovalbumin (OVA) to both legs of a six-week-old Std: Hartley male guinea pig on day 1 and day 8 after the initiation of sensitization, and intraperitoneally administering 1.5×10⁵ cells/mL/animal of pertussis vaccine.

### <Induction of Antigen>

On day 19 to day 23 after the first sensitization, the sensitized guinea pig was allowed to inhale a 2% OVA solution for five minutes to cause the antigen induction. Four hours after the induction, each organ was recovered.

### <Measurement of β-Glucuronidase Activity>

From three groups of a group of unsensitized guinea pigs, a group of sensitized guinea pigs, and an antigen induced group of the sensitized guinea pigs (two individuals in each group), each organ was removed, added with physiological saline in an amount 50 times the volume of the organ, homogenized, and subjected to cold centrifugation at 4°C at 12,000 rpm for 10 minutes to obtain a supernatant liquid as the sample. The β-glucuronidase activity in each sample was measured by colorimetry of a liberated p-nitrophenol at 405 nm with the use of p-nitrophenyl-β-D-glucuronide as the substrate according to the conventional method (Haeberlin et al., Pharmaceutical Res., 10, 1553-1562, 1993). The protein concentration in each sample was measured with the use of a commercially available kit. The specific activity of β-glucuronidase was shown as the mass of the reaction product to be liberated per mg of the protein per min. The specific activity of each organ was shown by the mean value

### 2. Result

The β-glucuronidase activity in each organ of three groups of a group of unsensitized guinea pigs, a group of sensitized guinea pigs, and an antigen induced group of sensitized guinea pigs were shown in Fig. 2. The β-glucuro-nidase activities in each organ of a group of unsensitized guinea pigs showed high values in the lungs (15 nmol/mg/min), the liver (20.7 nmol/mg/min), and the spleen (14.2 nmol/mg/min) and low values in the heart (1.9 nmol/mg/min), the brain (2.6 nmol/mg/min), and the muscle (1.2 nmol/mg/min). This result showed the same tendency as the reports on the enzymatic activities in each organ of rats, mice and the like (Conchie et al., Biochem. J. 71, 318-325, 1959, Johnson et al., Biochemical Genetics 24, 891-909, 1986, and Hoogerfrugge et al., Transplantation 43, 609-614, 1987) heretofore having been reported.

On the other hand, in the group of the sensitized guinea pigs, no difference of the enzymatic activities in each organ has been recognized in comparison with those in the group of unsensitized guinea pigs. This result suggests that in the sensitized state the enzymatic activities in each organ are not affected as in the case of unsesitization. Furthermore, no remarkable difference in the enzymatic activities in each organ even in the antigen induced group of sensitized guinea pigs has been recognized in comparison with those in the group of unsensitized guinea pigs and in the antigen noninduced group of sensitized guinea pigs.

It has been confirmed from these results that the lungs are the tissue having a very high β-glucuronidase activity and the β-glucuronidase in the lung tissue does not increase in the asthmatic model by antigen sensitization.

### [Reference Example 4]

### <<Fig. 3: Localization of β-Glucuronidase in Heart>>

### 1. Test Method

The test method is the same as described in «Fig. 1: Localization of β-Glucuronidase in Lungs». The cell nuclei were stained with hematoxylin as the counterstain.

### 2. Result

A frozen section of the heart was prepared, and the result of the vital staining of the tissue by the β-glucuronidase activity is shown in Fig. 3. As shown in Fig. 3, in the section of the heart of guinea pigs, no positive image showing the β-glucuronidase activity was observed.

### INDUSTRIAL APPLICABILITY

The present invention can provide a prodrug capable of reducing side effects of a drug on non-target organs by utilizing an enzymatic activity having a difference of the activity in between the target site of the drug and the site to express side effects. The present invention has enabled the provision of β₂-agonists which do not express side effects on the heart. Furthermore, the present invention has enabled the usage of a β₂-agonists to a patient who has a heart disease which restrain the usage of a β₂-agonists.

## Claims

1. A compound of formula (1)
R-Drug (1)
(wherein R means a substituent cleavable with an enzyme whose activity is different in between the target site of Drug and the site to express side effects; and Drug is a drug to be activated by cleaving the substituent with the enzyme) or its physiologically acceptable salt.

2. A compound of formula (2)
R'-Drug (2)
(wherein R' means a substituent cleavable with β-glucronidase existing at a high activity in respiratory organs and at a low activity in the heart; and Drug means a drug for respiratory organs to be activated by cleaving the substituent with the enzyme) or its physiologically acceptable salt.

3. The compound of claim 1, wherein the target site is a respiratory organ.

4. The compound of claim 1, wherein the site to exhibit side effects is the heart.

5. The compound of claim 1 or 2, wherein the Drug is a bronchodilator.

6. The compound of claim 1 or 2, wherein the Drug is a β₂-agonist.

7. The compound of claim 1 or 2, wherein the Drug is a β₂-agonist which has a hydroxyl group in its structure.

8. The compound of claim 1 or 2, wherein the Drug is any one of salbutamol, salmeterol, mabuterol, clenbuterol, pirbuterol, procaterol, fenoterol, tulobuterol, formoterol, hexoprenaline, terbutaline, trimetoquinol, chlorprenaline, orciprenaline, methoxyphenamine, methylephedrine, ephedrine, and isoprenaline.

9. The compound of claim 1, wherein the enzyme is a glycosidase.

10. The compound of claim 1, wherein the enzyme is β-glucuronidase.

11. The compound of claim 1 or 2, wherein the substituent is a glycosyl group of a monosaccharide or an oligo-saccharide.

12. The compound of claim 1 or 2, wherein the substituent is a glucuronyl group.

13. The compound of claim 11, wherein the bond between the glucuronyl group and the Drug is a β-bond.

14. A pharmaceutical composition comprising an effective amount of the compound of claim 1 or 2 together with pharmaceutically appropriate and physiologically acceptable fillers, additives and/or other active compounds and auxiliaries.

15. A pharmaceutical composition for local administration comprising an effective amount of the compound of claim 1 or 2 together with pharmaceutically appropriate and physiologically acceptable fillers, additives and/or other active compounds and auxiliaries.

16. A pharmaceutical composition for inhalation comprising an effective amount of the compound of claim 1 or 2 together with pharmaceutically appropriate and physiologically acceptable fillers, additives and/or other active compounds and auxiliaries.

17. Use of a compound to be represented by formula (1)
R-Drug (1)
(wherein R means a substituent cleavable with an enzyme whose activity is different in between the target site of Drug and the site to express side effects; and Drug is a drug to be activated by cleaving the substituent with the enzyme) or its physiologically acceptable salt for preparing a pharmaceutical composition which is a prodrug utilizing the enzyme whose enzymatic activity is different in between the target site of the Drug and the site to express side effects and expressing the effect in the target site alone.

18. Use of a compound to be represented by formula (2)
R'-Drug (2)
(wherein R' means a substituent cleavable by β-glucronidase existing at a high activity in respiratory organs and at a low activity in the heart; and Drug is a drug for respiratory organs to be activated by cleaving the substituent with the enzyme) or its physiologically acceptable salt for preparing a pharmaceutical composition which is a prodrug utilizing the enzyme whose enzymatic activity is different in between the target site of the Drug and the site to express side effects and expressing the effect in the target site alone.

19. The use of claim 17, wherein the target site is a respiratory organ.

20. The use of claim 17, wherein the site to exhibit side effects is the heart.

21. The use of claim 17 or 18, wherein the Drug is a bronchodilator.

22. The use of claim 17 or 18, wherein the Drug is a β₂-agonist.

23. The use of claim 17 or 18, wherein the Drug is a β₂-agonist which has a hydroxyl group in its structure.

24. The use of claim 17 or 18, wherein the Drug is any one of salbutamol, salmeterol, mabuterol, clenbuterol, -pirbuterol, procaterol, fenoterol, tulobuterol, formoterol, hexoprenaline, terbutaline, trimetoquinol, chlorprenaline, orciprenaline, methoxyphenamine, methylephedrine, ephedrine, and isoprenaline.

25. The use of claim 17, wherein the enzyme is a glycosidase.

26. The use of claim 17, wherein the enzyme is β-glucuronidase.

27. The use of claim 17 or 18, wherein the substituent is a glycosyl group.

28. The use of claim 17 or 18, wherein the substituent is a glycuronyl group.

29. The use of claim 28, wherein the bond between the glucuronyl group and the Drug is a β-bond.

30. A method for preparing a β₂-agonist having a sugar as a substituent which comprises reacting a β₂-agonist having a hydroxyl group with a sugar halide derivative in any one of solvents of acetone, acetonitrile, dioxane, and tetrahydrofuran in the presence of a base and deprotecting the resulting product by alkali hydrolysis.

31. The method of claim 30, wherein the base is either sodium hydroxide or potassium hydroxide.

32. A method for preparing a β₂-agonist having a sugar as the substituent which comprises adding a benzyl halide derivative to a mixture containing a β₂-agonist having a plurality of hydroxyl groups and a base to selectively protect the hydroxyl groups, then conducting glycosylation, subjecting the resulting intermediate to alkali hydrolysis, and then conducting hydrogenation.
